# EUROPEAN PATENT SPECIFICATION

(11) **EP 2 197 493 B1**
(45) Date of publication and mention of the grant of the patent: **29.11.2017**
(21) Application number: 08840644.2
(22) Date of filing: 14.10.2008
(51) Int. Cl.: B01J 13/02, A61K 9/50, C09B 67/02, A61K 47/42, A23J 1/20, A23J 3/08

(54) **MICROENCAPSULATED LIPOSOMAL COMPOSITIONS**
MIKROVERKAPSELTE LIPOSOMALE ZUSAMMENSETZUNGEN
COMPOSITIONS LIPOSOMALES MICROENCAPSULÉES

(30) Priority: 19.10.2007 FI 20075739
(43) Date of publication of application: 23.06.2010
(73) Proprietor: Biomed OY, 00940 Helsinki (FI)
(72) Inventor: MAHLBERG, Kaj, FI-00100 Helsinki (FI); SAVOLAINEN, Jouko, FI-02750 Espoo (FI); HILTUNEN, Raimo, FI-00180 Helsinki (FI); JAAKKOLA, Kaarlo, FI-00140 Helsinki (FI)
(74) Representative: Boco IP Oy Ab
(86) International application number: PCT/FI2008/050571
(87) International publication number: WO 2009/050333

(56) References cited:
- WO-A1-2005/036976
- WO-A1-2005/036977
- WO-A1-2006/115420
- WO-A1-2007/064225
- WO-A2-2004/112494
- WO-A2-2005/115341
- WO-A2-2006/033985
- WO-A2-2008/066380
- US-A- 5 601 760
- JIMENEZ M. ET AL.: 'Spray-dried encapsulation of Conjugated Linoleic Acid (CLA) with polymeric matrices' JOURNAL OF THE SCIENCE OF FOOD AND AGRICULTURE vol. 86, no. 14, 2006, pages 2431 - 2437, XP002458031

## Description

### Technical field

The invention relates to the field of liposomal compositions and particularly to microencapsulated liposomal compositions, for use as such, or as carriers and vehicles as well as formulations and compositions for the delivery of biologically active agents, such as nutritional supplements, herbs and plant extracts as well as pharmacologically active substances, such as drugs and hormones, for local or systemic action.

### State of the art

Food and other essential substances and agents like vitamins and minerals are obtained upon oral ingestion. Typically nutritional supplements and pharmaceutically active substances are provided in solid dosage forms, such as coated tablets, compressed tablets, compressed capsules and gelatine capsules, which are generally easy and relatively inexpensive to produce, readily dispensable and fairly stable. The dosage form influences the absorption and bioavailability of a biologically active compound.

Solid dosage forms comprising active agents, such as drug substances and nutritional supplements are in many cases not the optimal form for administering active substances because several compounds are degraded in the stomach. Degradation by the stomach acids decreases the therapeutic response to orally administered solid agents. Typically higher concentrations of active substances are needed in order to maintain the effectiveness of drugs and nutritional supplements, resulting in the increase of undesired side effects.

The activity of an orally administered substance mainly depends on the amount of the substance that reaches the bloodstream and the rate at which it reaches the bloodstream. The activity of the substance can be enhanced by increasing the rate and extent of absorption, thus reducing the amount of the active substance needed in the dosage form. The increase of the availability of a substance has become increasingly important and many solutions have been proposed in the art.

It is generally known that the rate of absorption and the extent or ratio of the amount of active material absorbed over the amount administered depends on dosage form and delivery system, physicochemical properties of the active material, such as solubility, dose, site of administration, vascularization of the absorption site, contact time with the absorption surface and pH at the site of absorption.

Solid dosage forms are absorbed following oral administration by a two-step process comprising dissolution of the solid dosage form and absorption of the active compound in solution.

The term "first-pass effect" is used to characterize the fraction of the active compound that is metabolized during the first exposure to the gut wall and the liver. All orally administered compounds that are absorbed from the gastrointestinal tract go to the portal vein and the liver before entering the systemic circulation. This means that, before an active compound that has been absorbed across the membrane of the gastrointestinal mucosa can reach the general circulation, it has to pass through the liver.

Polar lipids have a lipophilic and a hydrophilic group on the same molecules and upon interaction with water they self-assemble and form colloidal particles. Liposomes are lipid containing spherical particles. They can have one, several or many membranes. Liposomes are widely used as delivery vehicles for active agents in pharmaceutics, cosmetics, food and nutrition industry, coating industry and ecology. They enhance active substance solubilization, protect sensitive active molecules and enhance intercellular uptake, alter pharmacokinetics and biodistribution.

Several liposomal products enhancing the uptake or facilitating the delivery of various substances are known in the art. Parenteral and topical uses of liposomal carriers are disclosed in "Liposome Technology", 2nd Ed, Vol. I (1993) G. Gregoriadis ed., CRC Press, Boca Raton, Fla. Liposomal carriers protect a drug against hostile environments and provide controlled release of the drug while circulating in the blood or after immobilization at a target tissue such as the skin.

US 4,762,720 teaches liposome compositions capable of retaining larger amounts of drugs with a small amount of phospholipids, comprising freeze-dried MLV (multilamellar vesicles) or SUV (small unilamellar vesicles) in an aqueous medium, in the presence of an active ingredient and a divalent cation to regenerate LUV (large unilamellar vesicles) entrapping said active ingredient.

Various problems relate to orally administered liposomes. Low pH of the stomach and the presence of bile salts and lipases tend to destabilize the active substance - liposome complex. The stability can be increased using more stable bilayers, multilamellar liposomes and encapsulation into various capsules.

Liposomes are highly susceptible to destruction via uptake by the reticulo-endothelial system (RES), also known as the macrophage system, which can result in under-utilization and wastage of the entrapped active substance. A way of protecting liposomes from the RES and regulating the release profile is to encapsulate the drug-containing liposomes in a polymer matrix, in the form of a microcapsule. Such "microencapsulated liposome" systems have been studied for different purposes including for release of bioactive molecules, as described in Dhoot, N.O., Wheatley, M.A., 2003. Microencapsulated Liposomes in Controlled Drug delivery: Strategies to Modulate Drug Release and Eliminate the Burst Effect. J Pharm Scieces, Vol. 92, No. 3. Liposomes composed of a wide range of phospholipids such as phosphatidylchloline (PC), phosphatidylglycerol (PG), and dipalmitoyl phosphatidylcholine have been used in such systems, in conjuction with polymers used in microencapsulation, such as alginate, acacia-gelatin, gum arabic and gelatine alone.

Microencapsulated liposome systems have been shown to extend *in vivo* drug retention times and also elicit higher immune responses than liposomes alone. Typically, Ca²⁺ was used as the cross-linking ion with liposomes encapsulated in alginate, which is a water-soluble polysaccharide. A rapid initial burst of the active compound release was observed with liposomes encapsulated with Ca-alginate and Al-alginate coat. The rate of release of the drug can be modified by changing the composition of the lipid bilayer and by altering the concentration of the alginate, respectively. The coating of the alginate microcapsules with a rate controlling, size-selective membrane of cationic polypeptides such as poly-L-lysine and poly-L-ornithine increases the potential of the system. Typically microencapsulated products manufactured using alginate have an average particle size of around 20 µm and the manufacturing method utilizes temperatures of approximately 90 °C, precipitation, the use of Ca or A1 ions and an electrical field.

Also gum Arabic or a combination of gelatine and gum Arabic has been proposed for microencapsulation, however this combination typically needs an enzymatic or chemical treatment with glutaraldehyde to reinforce the bonding. Gum Arabic is able to encapsulate only the amount corresponding to its own weight of an active compound at most.

The phospholipids used in liposomal products typically originate from plants, mainly soy, and from eggs. The composition of plant-derived phospholipids differs significantly from human phospholipids since they contain neither cerebrocides nor sphingomyelines and phospholipids derived from eggs correspond to the phospholipids of birds. They are metabolised in the liver, thus releasing the active substances.

Whey is a common by-product in dairy industry, particularly cheese production and milk industry. Whey contains whey proteins, which structurally belong to the group of globulins, typically β-lactoglobulin as the major component, about 50-60 wt% of the protein content, α-lactalbumin, bovine serum albumin and immunoglobulins. Corresponding proteins in activity are also obtainable synthetically using suitable methods based on the addition of sulfhydryl groups to e.g. casein, but they are not food grade.

Native whey proteins have been suggested as microencapsulating agents for several substances and for example US 4,790,998 discloses a method for producing microcapsules from native whey protein concentrates as emulsifying agents and lipophilic substances, such as citrus oil. In this method microcapsules with a mean diameter of 1 µm were produced using heating at 75 °C. However, there are several problems relating to the use of native whey proteins.

Functional properties of whey proteins can be improved by modifying the protein molecules by sulfitolysis as described in US 6,797,810. The modified whey proteins contain typically two to four free SH-groups whereas the unmodified whey proteins contain on average less than one. The modification results in several advantageous properties like improved formation of protein network, emulsion and gel, as well as improved digestibility and antioxidant capacity. The modified whey proteins are suitable for applications in pharmaceutical and food industry as described in WO 2005/036977.

Based on the above it can be seen that there is need for alternative and improved liposomal compositions and carriers and vehicles for the delivery of active substances, such as drug substances and nutritional supplements and the like for the administration to a subject by suitable means.

### Definitions

The term availability of an active substance means here the completeness of absorption of an active substance.

The term bioavailability means the clinical description of availability *in vivo* and indicates the extent to which a substance reaches the bloodstream. Bioavailability is defined as the fraction per percentage of the administered dose that is ultimately absorbed intact.

### Summary of the invention

The present invention provides microencapsulated liposomal compositions, useful as such or as carriers and vehicles, as well as formulations and compositions for the delivery of biologically active agents, such as nutritional supplements, herbs and plant extracts as well as pharmacologically active substances, such as drugs and hormones, for local or systemic action. The liposomal compositions are microencapsulated with modified whey proteins. The biologically active agents include purified components such as drugs and hormones, nutritional supplements, vitamins and minerals, as well as semi-purified components such as plant extracts and any combinations thereof. The invention relates to microencapsulated liposomal compositions comprising liposomes and modified whey protein mixtures comprising whey protein concentrate and/or whey protein isolate and modified whey protein concentrate and/or modified whey protein isolate, and the liposomes comprise a lipid composition originating from bovine animals and/or pigs as disclosed in claim 1. The microencapsulated liposomal compositions, carriers, vehicles and formulations of the present invention comprise liposomes comprised of biologically active lipids obtainable from brain or spinal marrow or spinal cord of bovine animals, particularly of deers like reindeers, or pigs, the lipids being for example lecithin, ceramides, phosphatidylethanolamine, phosphotidylcholine, phosphatidylserine, cardiolipin as disclosed in table 1, microencapsulated with modified whey protein.

The liposomes may be of a variety of types, MLV (multilamella vesicles) or SUV (small unilamella vesicles) or LUV (large unilamella vesicles). One or more biologically active agents may be entrapped in the liposomal compositions, preferably in the liposomes.

The microencapsulated liposomal compositions of the present invention comprise powder with a dry matter composition of more than 95%, having an average particle size ranging from 0.1 to less than 10 µm, preferably 0.5 - 3 µm. It may be used for oral administration as tablets, capsules, suspensions, granules or powders, in aerosols or sprays as fine powder using known aerosol or pump spray delivery devices including coarse liquid sprays, aerosols of colloidal suspensions of liquid droplets in a gaseous carrier such as oxygen, nitrogen or a hydrocarbon propellant, and temporary suspensions of liquid droplets in the carrier, for parenteral administration, sublingual administration, buccal administration, nasal administration and local administration as ointments, gels and creams for subsequent local or systemic action.

The present disclosure further exemplifies methods for administering biologically active lipids and particularly phospholipids to a human or animal subject, comprising administering the microencapsulated liposomal composition according to the invention.

The present disclosure further exemplifies methods for administering biologically active agents, such as drugs and nutritional supplements, to a human or animal subject, comprising administering the microencapsulated liposomal composition containing at least one biologically active agent, according to the invention.

The present invention further provides a method for the manufacture of the microencapsulated liposomal compositions, carriers and vehicles as well as formulations comprising biologically active agents, such as drugs or nutritional supplements, for administering to a subject.

### Detailed description of the invention

The present invention provides microencapsulated liposomal compositions, carriers and vehicles as well as formulations, methods for the manufacture thereof and methods for administering them. Specifically, the present invention provides compositions for administering biologically active lipids and optionally other biologically active agents, such as drugs, hormones and nutritional supplements and the like. The microencapsulated liposomal compositions comprise biologically active lipids and optionally one or more biologically agents, microencapsulated with modified whey proteins. The present disclosure further provides examples of administering microencapsulated liposomal compositions comprising biologically active lipids and other biologically active agents. The compositions, carriers, vehicles and formulations and methods of the present invention provide improvements over conventional means previously used, by increasing the rate of absorption and bioavailability of the biologically active agent and in many cases also by decreasing the amount of the biologically active agent that is administered.

The microencapsulated liposomal composition, carrier, vehicle or formulation according to the invention comprises liposomes, according to claim 1, such as small unilamellar vesicles (SUV) or large unilamellar vesicles (LUV) or multilamellar vesicles (MLV) or oligolamellar vesicles or any combinations thereof. In the liposomes, the lipid bi-layers are made up mainly of phospholipids, which are amphiphilic; they have a hydrophilic head and a lipophilic tail. In aqueous solution the lipids are arranged into layers that form closed vesicles, like artificial cells.

The liposomes, which may also act as the liposomal encapsulating agent of the biologically active agent in the present invention, comprise biologically active lipids originating from bovine animals or pigs. The liposomes are microencapsulated with modified whey protein.

The biologically active lipids comprise a lipid composition of claim 1, forming the lipid vesicles and liposomes, comprising lipids naturally occurring in bovine animals or in pigs, or corresponding synthetically made lipids. The lipid composition is obtainable from brain or spinal marrow or spinal cord of bovine animals or pigs. Preferably the lipids originate from deer animals, such as reindeers or pigs. It is important that the lipids originate from mammals, particularly bovine animals and pigs, because their phospholipid compositions are essentially similar to human phospholipid composition.

The lipids are obtainable using suitably methods comprising extraction. In the method for the producing of a crude lipid mixture, suitable for the microencapsulated liposomal composition according to the invention, brain and/or spinal marrow and/or spinal cord originating from bovine animals, particularly deers and/or pigs is extracted with organic solvents and the solvents are evaporated. Preferably the brain and/or spinal marrow/spinal cord material is frozen, defrosted, ground, fried, cooled, lyophilised, macerated with a mixture of ethanol-diethylether and filtered. The filtrate is evaporated and the residue forms the crude lipid mixture. The crude lipid mixture comprises phospholipids and glycolipids derived from bovine animals and pigs.

The lipid composition (crude lipid mixture), of the present invention has typically the composition presented in following Table 1.

**Table 1.**

| **Component** | **Amount /% by weight** |
|---|---|
| Lysophosphatidylcholine | 0 - 1 |
| Sphingomyeline | 5 - 8 |
| Phosphatidylcholine | 7 - 15 |
| Lysophosphatidylethanolamine + phosphatidyl-L-serine | 2 - 4 |
| Phosphatidic acid | 0 - 1 |
| Phosphatidylethanolamine | 10 - 20 |
| Sulphatides | 1 - 3 |
| Cerebrocides | 10 - 25 |
| Cholesterol | 25 - 35 |
| Triglycerides | 10 - 25 |
| **Total amount of phospholipids and glycolipides** | 40 - 65 |

The lipid composition originates from bovine animals and/or pigs and it comprises 0 - 1 wt% of lysophosphatidylcholine, 5 - 8 wt% of sphingomyeline, 7 - 15 wt% of phosphatidylcholine, 2 - 4 wt% of lysophosphatidylethanolamine and phosphatidyl-L-serine, 0 - 1 wt% of phosphatidic acid, 10 - 20 wt% of phosphatidylethanolamine, 1 - 3 wt% of sulphatides, 10 - 25 wt% of cerebrocides, 25 - 35 wt% of cholesterol and 10 - 25 wt% of triglycerides.

The modified whey protein mixture suitable for the present invention comprises 60 - 95 wt% of whey protein concentrate (WPC) or whey protein isolate (WPI) or a mixture thereof, and 5 - 40 wt% of modified whey protein concentrate (MWPC) or modified whey protein isolate (MWPI) or a mixture thereof, calculated based on protein basis. The amount of MWPC/MWPI in the mixture depends mainly on the different lipophilic compounds used in the emulsions.

The whey protein concentrate (WPC) comprises protein, lactose, fat, minerals and moisture (water) and it is obtainable from whey for example by using ultrafiltration technique and drying. Typically the whey protein concentrate, for example WPC 80 where the number denotes the protein percentage, comprises 80 wt% of protein, 4.7 wt% of lactose, 6.0 wt% of fat, 2.8 wt% of minerals and 5.0 wt% of moisture. Preferably whey protein concentrate (WPC) with protein content of 70-85, preferably of 75-80 wt% is used.

The whey protein isolate (WPI) is obtainable from whey for example by ion exchange absorption techniques and drying. Typically the whey protein isolate, WPI comprises 92 wt% of protein, not more than 0.2 wt% of lactose, not more than 0.2 wt% of fat, not more than 4.5 wt% of minerals and not more than 6.0 wt% of moisture. Preferably whey protein isolate with protein content of 90-95 wt% is used.

The modified whey protein concentrate and modified whey protein isolate are obtained by modifying whey proteins by sulfitolysis as described in US 6,797,810, to cleave 15 - 30 % of the total disulfide bonds of the whey proteins, resulting in 2-4 times more free sulfhydryl (SH-) groups available in the protein, however the protein composition remaining the same. Free sulfhydryl groups are determined using the method described in Beveridge, T., Toma, S.J. & Nakai, S. 1974, Determination of SH- and SS- groups in some food proteins using Ellman's reagent, J. Food Sci. 39, 49-51, and Chan, K-Y. & Wasserman, B.P. 1993, Direct colorimetric assay of free thiol groups and disulfide bonds in suspensions of solubilized and particulate cereal proteins, Cereal Chem. 70, 22-26. The modified whey protein concentrate (MWPC) has protein content of 75-85, preferably 79-82 wt% and the modified whey protein isolate (MWPI) has protein content of 89-95, preferably of 90-91 wt%.

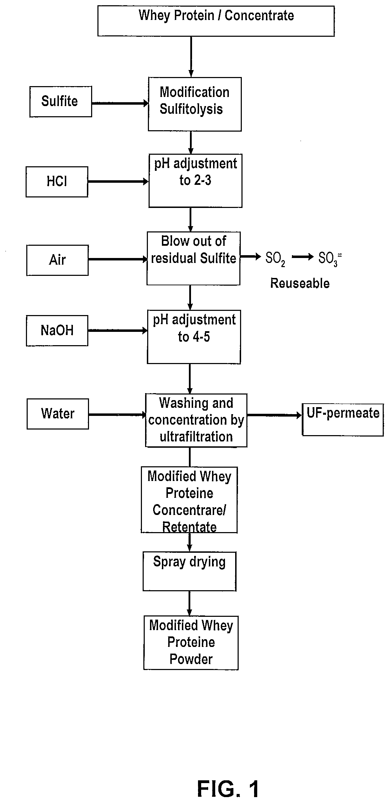

The process for the modification of whey proteins by sulfitolysis to obtain modified whey proteins is presented schematically in Figure 1.

In Figure 1 the production procedure for the manufacture of modified whey proteins is demonstrated. Whey protein concentrate in an aqueous solution, having 10-11 % (w/v) protein content and pH being 6-7, is warmed to a temperature of 50°-60°C. Then sulfite is added as 0.01-0.06 % (w/v) of sodium metabisulfite and the sulfonation /sulfitolysis reaction time is approx. 30 min. Thereafter the pH is adjusted with HCl to 2-3 to liberate SO₂ from the sulfonate derivatives of the protein and residual sulfite. The residual SO₂ is blown with air out of the reactor and it is reused as sulfite. Then the pH is adjusted to 4-5 with NaOH and the obtained protein concentrate is washed with water and ultrafiltered to the concentration of 10 - 30 % on protein content. The modified whey protein concentrate/retentate is spray-dried to modified whey protein powder at inlet and outlet temperature of 160°C and 80°C, respectively. Modified whey protein isolate is obtainable in a similar manner from whey protein isolate.

The modified whey protein mixture is formed of whey protein concentrate or isolate and modified whey protein concentrate or isolate or a mixture thereof, containing 60 - 95, preferably 70 - 90 wt% of the whey protein concentrate and/or isolate and 5 - 40, preferably 10 - 30 wt% of the modified whey protein concentrate and/or isolate. A method for the manufacture of a microencapsulated liposomal composition is exemplified as comprising the steps where modified whey protein mixture containing 60 - 95, preferably 70 - 90 wt% of whey protein concentrate and/or isolate and 5 - 40, preferably 10 - 30 wt% of modified whey protein concentrate and/or isolate, and having total protein content of 5 - 14, preferably 6 - 12 and particularly preferably 8 - 10 wt%, is dissolved with stirring in water having a temperature of 30° - 75°C, preferably 40° - 70°C, preferably sterile filtered or reverse osmosis (RO) treated tap water, which is particularly preferably filtered using a sterile filter with 0.1 µm pore size, followed by adding 7 - 15, preferably 8 - 14 wt% of the above defined lipid composition, as such or as a mixture with water of the same grade as above, comprising biologically active lipids originating from bovine animals and/or pigs (typically obtained as frozen crude lipid composition, which is defrosted before use), optionally containing 16 - 26, preferably 18 - 24 wt% of at least one biologically active agent dissolved or dispersed therein before the lipids are added to the mixture (or alternatively the biologically active agent is added to the lipid - water suspension obtained in the following step), the obtained mixture is agitated to form a lipid - water suspension, then the pH of the obtained suspension is adjusted to 5.5 - 8, preferably to 6 - 7 with an acid or base, preferably NaOH or HCl, the obtained suspension is heated to a temperature of 30° - 80°, preferably 45° - 70° and particularly preferably 50° - 60°C and dispersed or pre-homogenized with a high-shear mixer to form an emulsion, which is subjected to homogenisation using high pressure homogeniser under a pressure of 50 - 90, preferably 60 - 80 bar, then the emulsion is optionally pasteurised at a temperature of 65° - 85°, preferably 70° - 80 °C for 10 s to 5 min, preferably 10 s to 3 min or irradiated, then cooled to a temperature of 10° - 25°C and finally dried by spray drying or lyophilization, preferably in a spray dryer at an inlet temperature of 150° - 180°, preferably 155° -165°C and outlet temperature of 70° - 90°C, preferably 75° - 85°C using air or under an inert atmosphere, such as under nitrogen, to obtain a dry powdery product.

In the preparation of the emulsions the interchange reaction between WPC/WPI and MWPC/MWPI is essential to emulsification and formation of the emulsion. Heat treatment (preferably at 70°-80°C) enhances the completion of the reaction, and it is at the same time pasteurisation and reduces the amount of microbes. The heat treatment is applied after homogenisation and before drying.

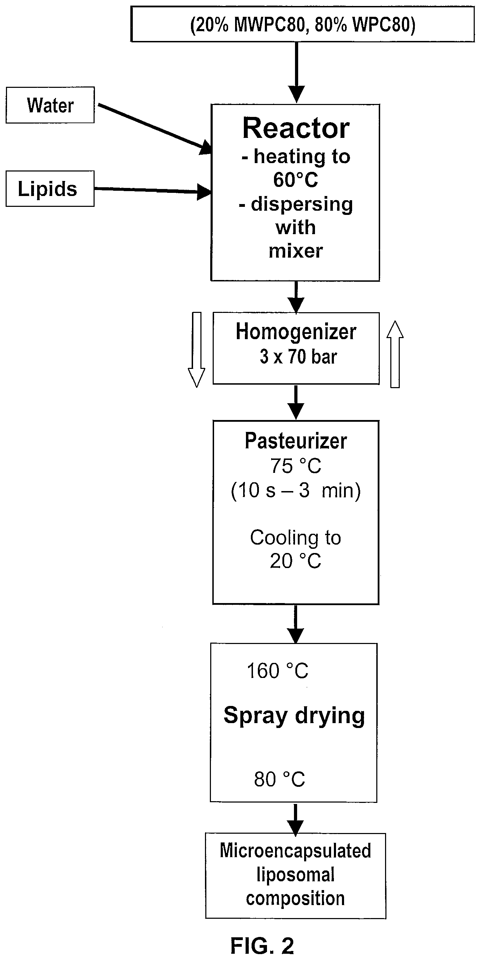

The method for the manufacture of the microencapsulated liposomal composition according to the invention is further illustrated with the flow chart presented in Figure 2, describing one preferable embodiment.

In Figure 2 modified whey protein mixture containing MWPC and WPC (ActiWhey®) having the total protein content of 60-80 g is dissolved in sterile filtered tap water, having a temperature of 30 - 75°C, with stirring, then 100-120 g of lipid composition is added and the mixture is agitated, then the pH of the obtained suspension is adjusted to 5.5 - 8, the obtained suspension is heated to a temperature of 60°C and dispersed or pre-homogenized with a high-shear mixer to form an emulsion, which is subjected to homogenisation using high pressure homogeniser under a pressure of 70 bar, (3x) then the obtained emulsion is pasteurised at a temperature of 75°C for 10 s to 3 min, cooled to 20°C and finally dried by spray drying in a spray dryer at an inlet temperature of 160°C and outlet temperature of 80°C, to obtain a dry powdery product.

The dry matter content of the obtained dry powdery product, the microencapsulated liposomal composition is more than 95 wt%. The microencapsulated liposomal composition according to the invention comprises particles having an average particle size from 0.1 to less than 10, preferably 0.5 - 3 µm.

During the pasteurisation the viscosity of the emulsion typically increases, and thus it is preferable to adjust the temperature and pasteurisation time such to keep the viscosity of the emulsion suitable for spray drying.

In the case heat sensitive active substances such as proteins or peptides are incorporated in the composition the product may be irradiated or an antimicrobial agent or agents may be added to the composition in amounts typically ranging from 0.001 - 1.0 wt% to prevent microbial contamination.

Antioxidants are preferably used in the composition in an amount of 0.001 - 0.5 wt% for the prevention of oxidation of the product. In both the wall/shell materials and core materials oxidation may start before the encapsulation, and thus it is preferable to stop and prevent any oxidation and also protect the product from oxidation during storage. Added antioxidants are efficient agents for these purposes. Suitable antioxidants are selected from the group consisting of ascorbic acid, tocopherols, ascorbyl palmitate, BHA, BHT and other antioxidants known in the art, as well as plant extracts having antioxidant power, such as rosemary extract. In the case water-soluble antioxidants are used they added to the whey protein mixture and oil-soluble antioxidants are in turn added to the lipids.

Additionally, a variety of further excipients and ingredients can be incorporated into the compositions, such as flavouring agents like orange oil, and colouring agents like carotenoids, as long as they do not excessively impair either the stability or bioavailability of the active agents contained within or associated with the liposome, in amounts typically ranging from 0.001 - 1.0 wt%.

One or more biologically active agents, intended to be administered to a subject, may be included in the composition according to the invention in an amount of 0.001 to 99.9 wt%. The biologically active agent is biologically active when the agent exerts a biological effect when administered to an organism. Suitable biologically active agents, which can be formulated in the present composition, include pharmaceutical agents such as drugs, hormones, proteins, peptides and nutritional supplements such as plant extracts, minerals, antioxidants and vitamins.

A nutritional supplement is defined as any substance that is administered as a dietary supplement to a subject. Any form of nutritional supplement that is capable of being entrapped in or bound to the lipid vesicle can be included in the compositions of the present invention. Suitable supplements include those both water-soluble and those that are fat-soluble, particularly antioxidants, vitamins and minerals, herbs and plant extracts. The preferred nutritional supplements are extracts of plants, particularly herbal plants, plant derived phenolic compounds, such as flavonoids, phenyl propanoids and the like. Examples of such nutritional supplements include, but are not limited to, Ginkgo biloba extract, Kava Kava extract, Ginseng extract, Saw Palmetto extract, glucosamine sulfate, chromium picolinate, Milk thistle extract, Grape seed extract, Ma Huang extract, Co-Q10 supplement, water soluble vitamins such as vitamin C, niacin, vitamins B like B1 and B12, fat soluble vitamins like vitamins A, D, E, and K. The preferred agents are those protecting nerve cells and blood vessels, such as antioxidants like Co-enzyme Q10.

The biologically active agents can be delivered as single agents, combinations of agents and combinations that might incorporate other desirable biologically active materials.

It is generally contemplated that the microencapsulated liposomal compositions agents according to the present invention can be administered orally as powders, granules, tablets, powder placed in gelatin capsules, suspension or other orally available formulations, in aerosols or sprays as powder using known aerosol or pump spray delivery devices including coarse liquid sprays, aerosols of colloidal suspensions of liquid droplets in a gaseous carrier such as oxygen, nitrogen or a hydrocarbon propellant, and temporary suspensions of liquid droplets in the carrier, parenterally, sublingually, as buccal administration, nasal administration and local administration as ointments, gels and creams, with appropriate excipients and modifiers known to those skilled in the art.

The liposomal composition according to the invention containing the biologically active lipids encapsulated with modified whey proteins provides improved means for effective administration of the lipids to a subject and prolonged effect. These lipids comprise phospholipids, which are essential building blocks and maintaining agents of cell membranes, particularly maintaining the fluidity of the cell membrane. Said lipids tend to seek there way particularly to neurological tissue and damaged sites. They also pass the blood-brain barrier and it has been demonstrated that they have positive effect on the congnitive functions such as memory, on cell repair and on cholesterol synthesis thus lowering serum cholesterol.

In the Examples that follow, the microencapsulated liposomal composition comprising Co-enzyme Q10 as the biologically active agent is described. Suitably CoQ10 (ubiquinone) can be administered to a subject in the microencapsulated liposomal composition according to the invention. The administering can be made less frequently in order to maintain an even dosing regimen in which blood levels remain steadier than would be the case with conventional preparations. In the Examples it can be seen that microencapsulated liposomal encapsulated CoQ10 according to the invention was found to provide a significant increase in the bioavailability of CoQ10, and further, the elimination was significantly slower, when compared to conventional CoQ10 formulation.

Notably the microencapsulated material itself need not be 100 % microencapsulated, and the microencapsulation rate can vary and be optimized within a range from about 0.1 to about 100 %, preferably 50 - 100 %,and particularly preferably 70 - 100 % being encapsulated.

As used herein, an agent is said to be lipid encapsulated when the agent is present within or on the surface of a lipid sphere. This includes, for example, entrapment within an enclosed lipid monolayer or bi-layer either by fusing smaller vesicles around the substance, transmission through the membrane of a formed lipid vesicle or liposome, forming the lipid vesicle or liposome within a solution containing the substance, or binding the substance or the lipid vesicle liposome membrane itself. Such formulations are representative of those known generally in the art.

Any subject can be administered the compositions of the present invention. Such subjects include humans as well as other mammalian organisms. It is essential to the invention that lipids originating from mammals are used. Agents, such as nutritional supplements, are becoming important components also in the diets of domesticated mammals such as pets and livestock.

As the microencapsulated liposomal composition according to the invention contains phospholipids originating only from mammals, no degradation of these compounds takes place in the liver of the mammal subject and the composition passes unaltered to the bloodstream. Thus the biologically active agents also remain intact until they reach the blood stream, resulting in that no material is lost due to degradation and the amount of the biologically active agent administered to a subject can be decreased. This results in that less active agent is needed for achieving the desired effect, less side effects and the compositions are more cost effective.

The microencapsulation method using modified whey protein mixtures makes it possible to influence the release site, way and rate of the active substances in the digestive tract and the release rate may be decreased by heat treatment.

### Examples

### Example 1

### Manufacture of crude lipid composition

Brain and spinal marrow material obtained from pigs was frozen and stored at a temperature of -18°C. Then it was defrosted at 22°C, ground with ethanol (96 %) for 2.5 hours, fried at a temperature of 110°C, cooled to room temperature and then frozen to a temperature of -28° - -32°C and lyophilised. The lyophilised product was heated to 35°C, crushed and stored at - 35°C. To 10 - 15 kg of the crushed product 20 1 of ethanol (96 %) and 5 1 of diethyl ether was added and the mixture was agitated/ macerated for 16 -20 hours and then filtered. The filtrate was evaporated and the obtained fatty residue (crude lipid composition) was frozen at -18°C.

### Example 2

### Manufacture of the microencapsulated liposomal composition containing ubiquinone

The modified whey protein mixture used for microencapsulation is described as follows: Modified whey protein mixture (ActiWhey®) containing 80 wt%, calculated based on protein content, of WPC 80 and 20 wt% of MWPC 80, that is modified by sulfitolysis as described in US 6,797,810 was used. The MWPC protein mixture contains 2-4 times more free sulfhydryl/SH groups available at the beginning of emulsification than the WPC at the same protein concentration.

The manufacture of microencapsulated product was carried out as follows:
1. 330 g of the modified whey protein mixture (Actiwhey®) was dissolved in 1.5 1 of (40°-50°C) warm RO-water (reverse osmosis) with stirring.
**2.** 330 g of the crude lipid composition manufactured in Example 1 was defrosted and added in 1.5 1 of (40°-50°C) warm RO-water with stirring to obtain a suspension.
3. 90 g of ubiquinone was suspended in the above obtained suspension of the lipid composition/phospholipids with stirring while protecting from light.
4. The modified whey protein solution obtained in step 1. and the lipid composition/phospholipid - ubiqinone suspension obtained in step 3. were mixed together with stirring to form a suspension.
5. pH of the suspension was adjusted to 6.5 with 1.0 M NaOH.
6. RO-water was added to the suspension and filled up to 3.75 1 volume.
7. The suspension was heated to 55°C and pre-homogenized with Ultra Turrax for 1 min. to get a coarse pre-emulsion.
8. The obtained pre-emulsion was passed through a FT-9 homogenizer four times at 70 bar.
9. Thereafter the emulsion was pasteurised at 70°C for 15 sec.
10. Then the emulsion was cooled to room temperature for the spray drying or to storage temperature of 6°C.

### Antioxidants

Ascorbic acid was used as antioxidant and it was added in an amount of 50 ppm in the modified whey protein solution (step 1).

### Spray-drying

The pasteurized and cooled emulsion was spray dried at inlet and outlet temperature of 160°C and 80°C, respectively. The dry substance content of the obtained microcapsules was 96-98 %.

### Analysis

Dry substance content of the microencapsulated powder was determined with IR device Scaltec SMO 01 and it was 97.1 wt%.

The fat globule shape and size/diameter of the emulsions and microencapsulated powders were photographed and measured with Olympus BX 60 Sony DXC- 950P Analysis 3.0.

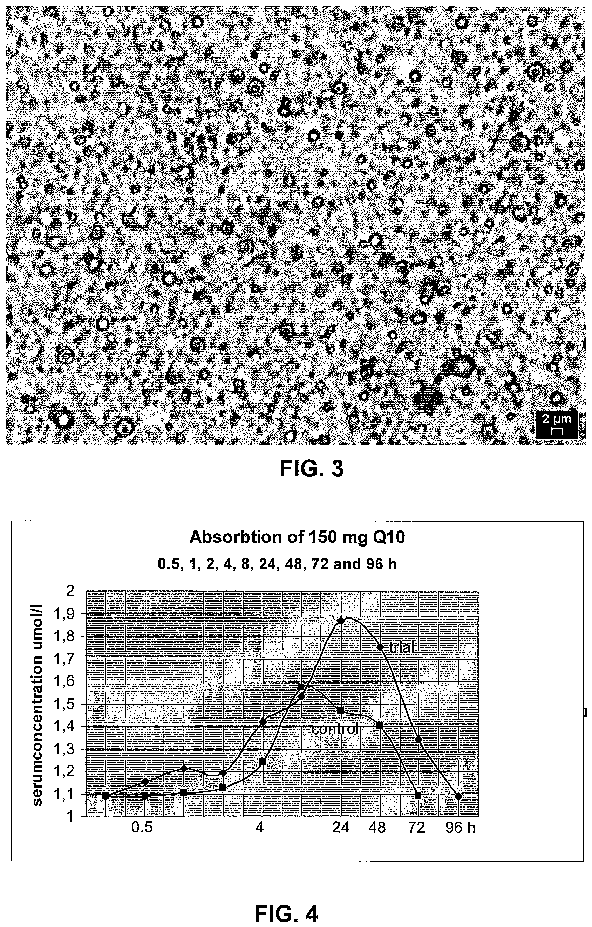

In Figure 3 it can be seen that the average particle size of the protein covered fat globules in emulsion was 1 µm or below and shape was separate and round globules.

### Example 3.

### Comparison of the efficacy of microencapsulated liposomal composition comprising ubiquinone with a traditional ubiquinone product for drug delivery

### Introduction

Controlled drug delivery can be achieved with natural or synthetic drug delivery devises in such a way that the active agent is released from the material in a predesigned manner. The aim is to release the active component over an extended period of time so that maximum therapeutic body fluid levels with a minimum of drug concentration can be maintained. Using controlled-delivery systems, the maintenance of drug levels within a desired range can be achieved. Nontoxic sustained drug delivery systems can also increase patient compliance with fewer administrations. Controlled drug delivery can optimize drug use.

The aim of this example was to assess the efficacy of the modified whey protein microencapsulated liposomal composition (biologically active carrier) for drug delivery. The microencapsulated liposomal composition (liposome) was prepared based on sterile phospholipids extracted from pig brains and spinal cords as described in examples 1 and 2. As a marker for drug delivery and intestinal absorption ubiquinone was used.

Serum concentrations achieved with the whey protein microencapsulated liposomal composition were compared with a commercial ubiquinone product (PharmaNord, soft gelatin capsule 150 mg) on the market.

Ubiquinone, a vitamin-like benzoquinone was chosen since it is present in all human cell mitochondria. Ubiquinone is essential for cellular energy production. Organs with high energy requirements such as the heart, the lungs and the liver have the highest ubiquinone concentrations.

Ubiquinone also serves as an important antioxidant in both mitochondria and lipid membranes and it is essential in vitamin E regeneration. The absorbed form of ubiquinone, ubiquinol inhibits protein and lipid oxidation in cell membranes, and helps to minimize oxidative injury to DNA. Endogenously produced free radicals are considered to be important factors in the aging process.

Ubiquinole production decreases with increasing age. Also cholesterol lowering drugs have been reported to decrease serum ubiquinone levels. Except for some rare medical conditions, increasing age, high cellular energy consumption and prevention of myopatic symptoms in patients treated with cholesterol lowering statins are indications for ubiquinone supplementation.

### Material and methods

Nine healthy volunteers participated in the modified trial. Serum samples for base line ubiquinone (Q10) determinations were taken before the trial. After a single ingestion of microencapsulated liposomal composition according to the invention, containing 150 mg of Q10, as a gelatin capsule, blood samples were drawn at regular intervals (0.5 h, 1 h, 2 h, 4 h, 8 h, 24 h, 48 h, 72 h, as presented in following Table 1).

After a wash-out period of two weeks five of the above volunteers participated in the control trial. The same amount of Q10 (150 mg) was taken as a single doze of the commercial ubiquinone product.

Serum of all the blood samples were analyzed in the same way. The serum samples were reduced with NaBH4, transforming the components of the sample to ubiquinole. Different fractions of the samples were separated by HPLC. A electrochemical detection which first oxidizes and once again reduces the samples was used. The results obtained are based on difference potentials caused by oxidation-reduction reactions (Nylander M et al. Plasma levels of coenzyme Q10 before and after oral supplementation: A bioavailability study. CoQ Res. Biol. Med. 1995; 3; 1: 25-32).

### Results

The results showed that the microencapsulated liposomal composition according to the invention, containing Q10, (=trial) achieved the highest Q10 concentration at 24 h (1.87 umol/l). Thereafter a decline was seen and the serum concentrations reached baseline at 96 h (1,09 umol/l).

The commercial product (=control) achieved the highest serum concentration at 8h (1,57 umol/l). Baseline Q10 serum levels were reached after 72 h.

In the attached Figure 4 the absorption of Q10 with trial and control products is presented as a function of serum concentration and time.

For the area under the curve calculations (AUC total), reflecting the bioavailability and the intestinal absorption of the drug, the trapezoidal-AUC method was used. The base line serum Q10 concentrations were subtracted from the samples (as can be seen in Table 1).

The calculated area under the curve for the microencapsulated liposomal composition according to the invention, containing 150 mg of Q10, was 43.10 µmol/L × h and for the control 20.34 µmol/L × h. The new Q10 liposomal drug delivery system increased serum concentrations with more then the double. The elimination time was also substantially longer for the microencapsulated Q10 product.

### Discussion

These results demonstrate that fat soluble ubiquinone, incorporated into whey protein microencapsulated liposomal composition can enhance ubiquinone absorption from the intestine. Since water and fat soluble drugs can be incorporated in whey protein microencapsulated liposomal composition, this novel drug delivery devise can be used for several different drugs. Using the whey protein microencapsulated liposomal composition, the amount of a drug can be reduced, without compromising patient treatment. Alternatively, longer interval between drug delivery can give better patient compliancy.

**Table 1: Q10 Study**

| **Trial composition** | | | | | | |
|---|---|---|---|---|---|---|
| **T (h)** | **C (µmol/L)** | **C - C _{basal level}** | **T₂-T₁** | **(C₁+C₂)/ 2** | **AUC _{(C1- >C2)}** | **AUC_{cumulative}** |
| 0 | 1.09 | 0 | | | | |
| 0.5 | 1.15 | 0.06 | 0.5 | 0.030 | 0.015 | 0.015 |
| 1 | 1.21 | 0.12 | 0.5 | 0.090 | 0.045 | 0.060 |
| 2 | 1.19 | 0.1 | 1 | 0.110 | 0.110 | 0.170 |
| 4 | 1.42 | 0.33 | 2 | 0.215 | 0.430 | 0.600 |
| 8 | 1.53 | 0.44 | 4 | 0.385 | 1.540 | 2.140 |
| 24 | 1.87 | 0.78 | 16 | 0.610 | 9.760 | 11.900 |
| 48 | 1.75 | 0.66 | 24 | 0.720 | 17.280 | 29.180 |
| 72 | 1.34 | 0.25 | 24 | 0.455 | 10.920 | 40.100 |
| 96 | 1.09 | 0 | 24 | 0.125 | 3.000 | 43.100 |
| | | | | | **AUC ₜₒₜₐₗ** | **43.10 µmol/L h** |

| **Control** | | | | | | |
|---|---|---|---|---|---|---|
| **T (h)** | **C (µmol/l)** | **C - C _{basal level}** | **T₂-T₁** | **(C₁+C₂)/ 2** | **AUC _{(C1- >C2)}** | **AUC _{cumulative}** |
| 0 | 1.09 | 0 | | | | |
| 0.5 | 1.09 | 0 | 0.5 | 0.000 | 0.000 | 0.000 |
| 1 | 1.10 | 0.01 | 0.5 | 0.005 | 0.003 | 0.003 |
| 2 | 1.12 | 0.03 | 1 | 0.020 | 0.020 | 0.023 |
| 4 | 1.24 | 0.15 | 2 | 0.090 | 0.180 | 0.203 |
| 8 | 1.57 | 0.48 | 4 | 0.315 | 1.260 | 1.463 |
| 24 | 1.47 | 0.38 | 16 | 0.430 | 6.880 | 8.343 |
| 48 | 1.4 | 0.31 | 24 | 0.345 | 8.280 | 16.623 |
| 72 | 1.09 | 0 | 24 | 0.155 | 3.720 | 20.343 |
| 96 | not measured | | | | | |
| | | | | | **AUCₜₒₜₐₗ** | **20.34 µmol**/**L h** |

## Claims

1. A microencapsulated liposomal composition comprising liposomes and a modified whey protein mixture comprising whey protein concentrate and/or whey protein isolate and modified whey protein concentrate and/or modified whey protein isolate, the liposomes comprise a lipid composition originating from bovine animals and/or pigs and the lipid composition comprises 0 - 1 wt% of lysophosphatidylcholine, 5 - 8 wt% of sphingomyeline, 7 - 15 wt% of phosphatidylcholine, 2 - 4 wt% of lysophosphatidylethanolamine and phosphatidyl-L-serine, 0 - 1 wt% of phosphatidic acid, 10 - 20 wt% of phosphatidylethanolamine, 1 - 3 wt% of sulphatides, 10 - 25 wt% of cerebrocides, 25 - 35 wt% of cholesterol and 10 - 25 wt% of triglycerides, the dry matter content of the microencapsulated liposomal composition is more than 95 wt% and the microencapsulated liposomal composition has average particle size from 0.1 µm to less than 10 µm.

2. The microencapsulated liposomal composition according to claim 1, **characterised in that** the dry matter content of the microencapsulated liposomal composition is more than 95 wt% and the average particle size is 0.5 - 3 µm.

3. The microencapsulated liposomal composition according to claim 1 or 2, **characterised in that** the modified whey protein mixture contains 60 - 95 wt% of whey protein concentrate and/or whey protein isolate and 5 - 40 wt% of modified whey protein concentrate and/or modified whey protein isolate, preferably the modified whey protein mixture contains 70 - 90 wt% of whey protein concentrate and/or whey protein isolate and 10 - 30 wt% of modified whey protein concentrate and/or modified whey protein isolate.

4. The microencapsulated liposomal composition according to any one of claims 1 - 3, **characterised in that** the microencapsulated liposomal composition comprises one or more biologically active agents selected from pharmaceutical agents and nutritional supplements, preferably the pharmaceutical agent is selected from drugs, hormones, proteins and peptides and preferably the nutritional supplement is selected form plant extracts, minerals, antioxidants and vitamins.

5. The microencapsulated liposomal composition according to any one of claims 1 - 4, **characterised in that** the microencapsulated liposomal composition comprises additives selected from antioxidants, antimicrobial agents and excipients.

6. A method for the manufacture of the microencapsulated liposomal composition according to any one of claims 1-5, **characterised in that** the method comprises the steps where a modified whey protein mixture comprising whey protein concentrate and/or conventional whey protein isolate and modified whey protein concentrate and/or modified whey protein isolate is dissolved in water, then a lipid composition comprising lipids originating from bovine animals and/or pigs, where said lipid composition comprises 0 - 1 wt% of lysophosphatidylcholine, 5 - 8 wt% of sphingomyeline, 7 - 15 wt% of phosphatidylcholine, 2 - 4 wt% of lysophosphatidylethanolamine and phosphatidyl-L-serine, 0 - 1 wt% of phosphatidic acid, 10 - 20 wt% of phosphatidylethanolamine, 1 - 3 wt% of sulphatides, 10 - 25 wt% of cerebrocides, 25 - 35 wt% of cholesterol and 10 - 25 wt% of triglycerides, is added and the mixture is agitated to form a suspension, the pH of the obtained suspension is adjusted to 5.5 - 8, and the suspension is heated to a temperature of 30 - 80°C and dispersed or pre-homogenized to form an emulsion, which is subjected to homogenisation under a pressure of 50 - 90 bar, the emulsion is optionally pasteurised at a temperature of 65 - 85°C, or irradiated, cooled and dried.

7. The method according to claim 6, **characterised in that** the modified whey protein mixture contains 60 - 95 wt% of whey protein concentrate and/or whey protein isolate and 5 - 40 wt% of modified whey protein concentrate and/or modified whey protein isolate, preferably the modified whey protein mixture contains 70 - 90 wt% of whey protein concentrate and/or whey protein isolate and 10 - 30 wt% of modified whey protein concentrate and/or modified whey protein isolate.

8. The method according claim 6 or 7, **characterised in that** the total protein content of the modified whey protein mixture is 5 - 14, preferably 6 - 12 wt%.

9. The method according to any one of claims 6 - 8, **characterised in that** the modified whey protein mixture is dissolved in water having the temperature of 30 - 75°C, preferably 40 - 70°C.

10. The method according to any one of claims 6 - 9, **characterised in that** 7 - 15, preferably 8 - 14 wt% of the lipid composition is added.

11. The method according to any one of claims 6 - 10, **characterised in that** one or more biologically active agent is dissolved or dispersed in the lipid composition before it is added to the modified whey protein mixture dissolved in water.

12. The method according to claim 11, **characterised in that** one or more biologically active agent selected from pharmaceutical agents and nutritional supplements is added to the suspension formed after the lipid composition is added to the modified whey protein mixture dissolved in water, preferably the pharmaceutical agent is selected from drugs, hormones, proteins and peptides and preferably the nutritional supplement is selected from plant extracts, minerals, antioxidants and vitamins.

13. The method according to any one of claims 6 - 12, **characterised in that** the pH of the suspension is adjusted to 5.5 - 8 with an acid or base.

14. The method according to any one of claims 6 - 13, **characterised in that** the product is dried by spray-drying or lyophilization.

15. The method according to any one of claims 6 - 14, **characterised in that** an additive selected from antioxidants, antimicrobial agents and excipients is added to the modified whey protein mixture or to the lipid composition.

## Patentansprüche

1. Mikroverkapselte liposomale Zusammensetzung umfassend Liposome und ein modifiziertes Molkenproteingemisch, welches Molkenproteinkonzentrat und/oder Molkenproteinisolat und modifiziertes Molkenproteinkonzentrat und/oder modifiziertes Molkenproteinisolat umfasst, wobei die Liposomen eine Lipid-Zusammensetzung mit Ursprung aus Rindern und/oder Schweinen umfassen und die Lipid-Zusammensetzung 0 - 1 Gew.-% Lysophosphatidylcholin, 5 - 8 Gew.-% Sphingomyelin, 7 - 15 Gew.-% Phosphatidylcholin, 2 - 4 Gew.-% Lysophosphatidylethanolamin und Phosphatidyl-L-Serin, 0 - 1 Gew.-% Phosphatidsäure, 10 - 20 Gew.-% Phosphatidylethanolamin, 1 - 3 Gew.-% Sulfatide, 10 - 25 Gew.-% Cerebroside, 25 - 35 Gew.-% Cholesterol und 10 - 25 Gew.-% Triglyceride umfasst, der Trockensubstanzgehalt der mikroverkapselten liposomalen Zusammensetzung mehr als 95 Gew.-% beträgt und die mikroverkapselte liposomale Zusammensetzung eine durchschnittliche Teilchengröße von 0,1 µm bis unter 10 µm aufweist.

2. Mikroverkapselte liposomale Zusammensetzung nach Anspruch 1, **dadurch gekennzeichnet, dass** der Trockensubstanzgehalt der mikroverkapselten liposomalen Zusammensetzung mehr als 95 Gew.-% beträgt und die durchschnittliche Teilchengröße 0,5 - 3 µm beträgt.

3. Mikroverkapselte liposomale Zusammensetzung nach Anspruch 1 oder 2, **dadurch gekennzeichnet, dass** das modifizierte Molkenproteingemisch 60 - 95 Gew.-% Molkenproteinkonzentrat und/oder Molkenproteinisolat und 5 - 40 Gew.-% modifiziertes Molkenproteinkonzentrat und/oder modifiziertes Molkenproteinisolat enthält, wobei das modifizierte Molkenproteingemisch bevorzugt 70 - 90 Gew.-% Molkenproteinkonzentrat und/oder Molkenproteinisolat und 10 - 30 Gew.-% modifiziertes Molkenproteinkonzentrat und/oder modifiziertes Molkenproteinisolat enthält.

4. Mikroverkapselte liposomale Zusammensetzung nach einem der Ansprüche 1 bis 3, **dadurch gekennzeichnet, dass** die mikroverkapselte liposomale Zusammensetzung ein oder mehrere biologisch wirksame Mittel gewählt aus pharmazeutischen Wirkstoffen und Nahrungsergänzungsmitteln umfasst, wobei der pharmazeutische Wirkstoff bevorzugt gewählt ist aus Arzneimitteln, Hormonen, Proteinen und Peptiden und das Nahrungsergänzungsmittel bevorzugt gewählt ist aus pflanzlichen Extrakten, Mineralstoffen, Antioxidantien und Vitaminen.

5. Mikroverkapselte liposomale Zusammensetzung nach einem der Ansprüche 1 bis 4, **dadurch gekennzeichnet, dass** die mikroverkapselte liposomale Zusammensetzung Zusatzstoffe gewählt aus Antioxidantien, antimikrobiellen Mitteln und Arzneiträgerstoffen umfasst.

6. Verfahren zur Herstellung der mikroverkapselten liposomalen Zusammensetzung nach einem der Ansprüche 1 bis 5, **dadurch gekennzeichnet, dass** das Verfahren die Schritte umfasst, in denen ein modifiziertes Molkenproteingemisch, welches Molkenproteinkonzentrat und/oder herkömmliches Molkenproteinisolat und modifiziertes Molkenproteinkonzentrat und/oder modifiziertes Molkenproteinisolat umfasst, in Wasser gelöst wird, wonach eine Lipid-Zusammensetzung mit Lipiden mit Ursprung aus Rindern und/oder Schweinen, die 0 - 1 Gew.-% Lysophosphatidylcholin, 5 - 8 Gew.-% Sphingomyelin, 7 - 15 Gew.-% Phosphatidylcholin, 2 - 4 Gew.-% Lysophosphatidylethanolamin und Phosphatidyl-L-Serin, 0 - 1 Gew.-% Phosphatidsäure, 10 - 20 Gew.-% Phosphatidylethanolamin, 1 - 3 Gew.-% Sulfatide, 10 - 25 Gew.-% Cerebroside, 25 - 35 Gew.-% Cholesterol und 10 - 25 Gew.-% Triglyceride umfasst, hinzugegeben wird und das Gemisch zur Ausbildung einer Suspension gerührt wird, der pH-Wert der erhaltenen Suspension auf 5,5 - 8 eingestellt wird und die Suspension auf eine Temperatur von 30 - 80 °C erwärmt wird und dispergiert oder vorhomogenisiert wird, um eine Emulsion auszubilden, die einer Homogenisierung bei einem Druck von 50 - 90 bar unterzogen wird, wobei die Emulsion bei einer Temperatur von 65 - 85 °C optional pasteurisiert oder bestrahlt, gekühlt und getrocknet wird.

7. Verfahren nach Anspruch 6, **dadurch gekennzeichnet, dass** das modifizierte Molkenproteingemisch 60 - 95 Gew.-% Molkenproteinkonzentrat und/oder Molkenproteinisolat und 5 - 40 Gew.-% modifiziertes Molkenproteinkonzentrat und/oder modifiziertes Molkenproteinisolat enthält, wobei das modifizierte Molkenproteingemisch bevorzugt 70 - 90 Gew.-% Molkenproteinkonzentrat und/oder Molkenproteinisolat und 10 - 30 Gew.-% modifiziertes Molkenproteinkonzentrat und/oder modifiziertes Molkenproteinisolat enthält.

8. Verfahren nach Anspruch 6 oder 7, **dadurch gekennzeichnet, dass** der Gesamtproteingehalt des modifizierten Molkenproteingemischs 5 - 14, bevorzugt 6 - 12 Gew.-% beträgt.

9. Verfahren nach einem der Ansprüche 6 bis 8, **dadurch gekennzeichnet, dass** das modifizierte Molkenproteingemisch in Wasser, das eine Temperatur von 30 - 75 °C, bevorzugt 40 - 70 °C aufweist, gelöst wird.

10. Verfahren nach einem der Ansprüche 6 bis 9, **dadurch gekennzeichnet, dass** 7 - 15, bevorzugt 8 - 14 Gew.-% der Lipid-Zusammensetzung zugegeben wird.

11. Verfahren nach einem der Ansprüche 6 bis 10, **dadurch gekennzeichnet, dass** ein oder mehrere biologisch wirksame Mittel in der Lipid-Zusammensetzung gelöst oder dispergiert wird, bevor es dem in Wasser gelösten modifizierten Molkenproteingemisch zugegeben wird.

12. Verfahren nach Anspruch 11, **dadurch gekennzeichnet, dass** der Suspension ein oder mehrere biologisch wirksame Mittel gewählt aus pharmazeutischen Wirkstoffen und Nahrungsergänzungsmitteln zugegeben wird, nachdem die Lipid-Zusammensetzung dem in Wasser gelösten modifizierten Molkenproteingemisch zugegeben wird, wobei der pharmazeutische Wirkstoff bevorzugt gewählt ist aus Arzneimitteln, Hormonen, Proteinen und Peptiden und das Nahrungsergänzungsmittel bevorzugt gewählt ist aus pflanzlichen Extrakten, Mineralstoffen, Antioxidantien und Vitaminen.

13. Verfahren nach einem der Ansprüche 6 bis 12, **dadurch gekennzeichnet, dass** der pH-Wert der Suspension mit einer Säure oder Base auf 5,5 - 8 eingestellt wird.

14. Verfahren nach einem der Ansprüche 6 bis 13, **dadurch gekennzeichnet, dass** das Produkt durch Sprühtrocknung oder Gefriertrocknung (Lyophilisation) getrocknet wird.

15. Verfahren nach einem der Ansprüche 6 bis 14, **dadurch gekennzeichnet, dass** dem modifizierten Molkenproteingemisch oder der Lipid-Zusammensetzung ein Zusatzstoff gewählt aus Antioxidantien, antimikrobiellen Mitteln und Arzneiträgerstoffen zugegeben wird.

## Revendications

1. Composition liposomale microencapsulée comprenant des liposomes et un mélange de protéines de lactosérum modifiées comprenant du concentré de protéines de lactosérum et/ou de l'isolat de protéines de lactosérum et du concentré de protéines de lactosérum modifiées et/ou de l'isolat de protéines de lactosérum modifiées, lesdits liposomes comprenant une composition lipidique originaire d'animaux bovins et/ou de porcs et ladite composition lipidique comprenant 0 - 1 % en poids de lysophosphatidylcholine, 5 - 8 % en poids de sphingomyéline, 7 - 15 % en poids de phosphatidylcholine, 2 - 4 % en poids de lysophosphatidyléthanolamine et phosphatidyl-L-sérine, 0 - 1 % en poids d'acide phosphatidique, 10 - 20 % en poids de phosphatidyléthanolamine, 1 - 3 % en poids de sulfatides, 10 - 25 % en poids de cérébrosides, 25 - 35 % en poids de cholestérol et 10 - 25 % en poids de triglycérides, la teneur en matière sèche de la composition liposomale microencapsulée étant supérieure à 95 % en poids et la composition liposomale microencapsulée ayant une taille moyenne de particules de 0,1 µm jusqu'à moins de 10 µm.

2. Composition liposomale microencapsulée selon la revendication 1, **caractérisée en ce que** la teneur en matière sèche de la composition liposomale microencapsulée est supérieure à 95 % en poids et la taille moyenne de particules est de 0,5 à 3 µm.

3. Composition liposomale microencapsulée selon la revendication 1 ou 2, **caractérisée en ce que** ledit mélange de protéines de lactosérum modifiées contient 60 - 95 % en poids de concentré de protéines de lactosérum et/ou d'isolé de protéines de lactosérum et 5 - 40 % en poids de concentré de protéines de lactosérum modifiées et/ou d'isolé de protéines de lactosérum modifiées, ledit mélange de protéines de lactosérum modifiées contenant préférablement 70 - 90 % en poids de concentré de protéines de lactosérum et/ou d'isolé de protéines de lactosérum et 10 - 30 % en poids de concentré de protéines de lactosérum modifiées et/ou d'isolé de protéines de lactosérum modifiées.

4. Composition liposomale microencapsulée selon l'une des revendications 1 à 3, **caractérisée en ce que** la composition liposomale microencapsulée comprend un ou plusieurs agents biologiquement actifs choisis parmi les agents pharmaceutiques et les compléments nutritionnels, ledit agent pharmaceutique étant préférablement choisi parmi les médicaments, les hormones, les protéines et les peptides et ledit complément nutritionnel étant préférablement choisi parmi les extraits de plantes, les minéraux, les antioxydants et les vitamines.

5. Composition liposomale microencapsulée selon l'une des revendications 1 à 4, **caractérisée en ce que** la composition liposomale microencapsulée comprend des additifs choisis parmi les antioxydants, les agents antimicrobiens et les excipients.

6. Procédé de fabrication de la composition liposomale microencapsulée selon l'une des revendications 1 à 5, **caractérisé en ce que** ledit procédé comprend les étapes consistant à dissoudre dans de l'eau un mélange de protéines de lactosérum modifiées comprenant du concentré de protéines de lactosérum et/ou de l'isolat de protéines de lactosérum classiques et du concentré de protéines de lactosérum modifiées et/ou de l'isolat de protéines de lactosérum modifiées, puis à ajouter une composition lipidique comprenant des lipides originaires d'animaux bovins et/ou de porcs, ladite composition lipidique comprenant 0 - 1 % en poids de lysophosphatidylcholine, 5 - 8 % en poids de sphingomyéline, 7 - 15 % en poids de phosphatidylcholine, 2 - 4 % en poids de lysophosphatidyléthanolamine et phosphatidyl-L-sérine, 0 - 1 % en poids d'acide phosphatidique, 10 - 20 % en poids de phosphatidyléthanolamine, 1 - 3 % en poids de sulfatides, 10 - 25 % en poids de cérébrosides, 25 - 35 % en poids de cholestérol et 10 - 25 % en poids de triglycérides, et à remuer le mélange pour former une suspension, ajuster la valeur pH de la suspension ainsi obtenue à 5,5 - 8 et chauffer la suspension à une température de 30 - 80 °C et disperser ou pré-homogénéiser la suspension pour former une émulsion qui est assujettie à une homogénéisation sous une pression de 50 - 90 bar, ladite émulsion étant, en option, pasteurisée à une température de 65 - 85 °C, ou irradiée, refroidie et séchée.

7. Procédé selon la revendication 6, **caractérisé en ce que** ledit mélange de protéines de lactosérum modifiées contient 60 - 95 % en poids de concentré de protéines de lactosérum et/ou d'isolé de protéines de lactosérum et 5 - 40 % en poids de concentré de protéines de lactosérum modifiées et/ou d'isolé de protéines de lactosérum modifiées, ledit mélange de protéines de lactosérum modifiées contenant préférablement 70 - 90 % en poids de concentré de protéines de lactosérum et/ou d'isolé de protéines de lactosérum et 10 - 30 % en poids de concentré de protéines de lactosérum modifiées et/ou d'isolé de protéines de lactosérum modifiées.

8. Procédé selon la revendication 6 ou 7, **caractérisé en ce que** la teneur totale en protéines du mélange de protéines de lactosérum modifiées est de 5 - 14, préférablement de 6 - 12 % en poids.

9. Procédé selon l'une des revendications 6 à 8, **caractérisé en ce que** le mélange de protéines de lactosérum modifiées est dissous dans de l'eau ayant une température de 30 - 75 °C, préférablement de 40 - 70 °C.

10. Procédé selon l'une des revendications 6 à 9, **caractérisé en ce que** 7 - 15, préférablement 8 - 14 % en poids de la composition lipidique est ajoutée.

11. Procédé selon l'une des revendications 6 à 10, **caractérisé en ce que** un ou plusieurs agents biologiquement actifs sont dissous ou dispersés dans la composition lipidique avant d'être ajoutés au mélange de protéines de lactosérum modifiées dissout dans de l'eau.

12. Procédé selon la revendication 11, **caractérisé en ce que** un ou plusieurs agents biologiquement actifs choisis parmi les agents pharmaceutiques et les compléments nutritionnels sont ajoutés à la suspension formée après que la composition lipidique a été ajoutée au mélange de protéines de lactosérum modifiées dissout dans de l'eau, ledit agent pharmaceutique étant préférablement choisi parmi les médicaments, les hormones, les protéines et les peptides et ledit complément nutritionnel étant préférablement choisi parmi les extraits de plantes, les minéraux, les antioxydants et les vitamines.

13. Procédé selon l'une des revendications 6 à 12, **caractérisé en ce que** la valeur pH de la suspension est ajustée à 5,5 - 8 avec un acide ou une base.

14. Procédé selon l'une des revendications 6 à 13, **caractérisé en ce que** le produit est séché par séchage par pulvérisation ou par lyophilisation.

15. Procédé selon l'une des revendications 6 à 14, **caractérisé en ce que** un additif choisi parmi les antioxydants, les agents antimicrobiens et les excipients est ajouté au mélange de protéines de lactosérum modifiées ou à la composition lipidique.
